# EUROPEAN PATENT SPECIFICATION

(11) **EP 3 949 830 B1**
(45) Date of publication and mention of the grant of the patent: **12.02.2025**
(21) Application number: 21188709.6
(22) Date of filing: 30.07.2021
(51) Int. Cl.: A61B 3/10, A61B 5/00, G01B 9/02

(54) **CONTROL SYSTEM FOR AN OCT IMAGING SYSTEM, OCT IMAGING SYSTEM AND METHOD FOR IMAGING**
STEUERUNGSSYSTEM FÜR EIN OCT-ABBILDUNGSSYSTEM, OPTISCHES ABBILDUNGSSYSTEM UND VERFAHREN ZUR ABBILDUNG
SYSTÈME DE COMMANDE POUR UN SYSTÈME D'IMAGERIE TOMOGRAPHIQUE À COHÉRENCE OPTIQUE (OCT), SYSTÈME D'IMAGERIE TOMOGRAPHIQUE À COHÉRENCE OPTIQUE (OCT) ET PROCÉDÉ D'IMAGERIE

(30) Priority: 05.08.2020 DE 102020120651
(43) Date of publication of application: 09.02.2022
(73) Proprietor: Leica Microsystems NC, Inc., Durham, NC 27703 (US)
(72) Inventor: Perng, John, 9000 St. Gallen (CH); Lynch, Eric, 27523 Apex (US)
(74) Representative: J A Kemp LLP

(56) References cited:
- JP-A- 2019 133 429
- KR-A- 20170 008 172
- US-A1- 2015 015 845
- US-A1- 2017 014 025

## Description

### Technical Field

The present invention essentially relates to a control system for an optical coherence tomography (OCT) imaging system for real-time imaging of a subject, which includes a tissue material of interest, to an OCT imaging system including such control system, and to a method for imaging a subject in real-time, using OCT.

### Background

Optical coherence tomography (in the following also called OCT, its typical abbreviation) is an imaging technique that uses low-coherence light to capture two- and three-dimensional images from within optical scattering media (e.g., biological tissue) with high resolution. It is, inter alia, used for medical imaging. Optical coherence tomography is based on low-coherence interferometry, typically employing near-infrared light. The use of relatively long wavelength light allows it to penetrate into the scattering medium.

A medical field of particular interest for OCT is ophthalmology, a branch of medicine related to (in particular human) eyes and its disorders and related surgeries. The ability to visualize the vitreous body of an eye (sometimes also just called "vitreous") during vitrectomy is critical to surgical success. The vitreous body is a transparent tissue and becomes slightly hazy with aging. Currently, the standard of care is to apply a white dye (typically triamcinolone) to stain the vitreous body. However, there is known toxicity related to the dye, and many surgeons prefer to use as little as possible or no dye at all when performing vitrectomy.

Document US 2017/014024 A relates to OCT for imaging subjects like eyes. Different amounts of conversion for converting an intensity distribution are used for different regions of the image like retina and vitreous body. Document JP 2019/133429 A relates to OCT for imaging subjects like embryos.

### Summary

According to the invention, a control system, an OCT imaging system and a method for imaging a subject with the features of the independent claims are proposed. Advantageous further developments form the subject matter of the dependent claims and of the subsequent description.

The present invention relates to a control system for an optical coherence tomography (OCT) imaging system for real-time imaging of a subject, the subject including a tissue material of interest. This subject, preferably, includes or is an eye, and the tissue material of interest, preferably, includes or is a vitreous body of the eye. The control system is configured to control the optical coherence tomography imaging system (the type of OCT to be used is, preferably, spectral domain OCT, as will also be described later) to scan the subject by means of optical coherence tomography for acquiring intensity scan data. Such scanning can include, in particular, performing a B-scan.

In OCT, areas of the sample or tissue that reflect back a lot of light will create greater interference than areas that do not. Any light that is outside the short coherence length will not interfere. This reflectivity profile is called an A-scan and contains information about the spatial dimensions and location of structures within the sample or tissue. A cross-sectional tomograph, called B-scan, may be achieved by laterally combining a series of these axial depth scans (A-scan).

In general, such intensity scan data is processed in order to provide image data than is to be or than can be visualized in display means, e.g., a display next to the OCT scanning system or the like. Due to the large range of intensity typically acquired during such scan, the image data is, up to now, determined based on a conversion scale that allows to condense the data such that all relevant intensity data can be visualized within a range of visible light. Typically, a conversion scale based on a logarithmic scale is used or such purposes.

A consequence of using such a conversion scale is that certain tissue material of interest like the vitreous body of an eye is not visible in image obtained for the image data. This is also the reason why staining the vitreous body was performed, as mentioned earlier.

It has now turned out that such tissue materials of interest (in a subject under imaging) like the vitreous body of an eye (and which is not visible without staining in OCT images up to now) can be made visible. In order to achieve this, within the present invention, when processing the intensity scan data (performed by the control system) to to provide the image data to be visualized on display means, for a selected range of intensity, correlated to the tissue material of interest, the image data is determined based on a selected conversion scale that is different from a conversion scale for intensity outside the selected range. While the conversion scale for intensity outside the selected range can, as previously, be based on a logarithmic scale, the conversion scale for the selected range is, preferably, based on a linear scale. Such different scale like or based in a linear scale allows much more of the intensity range of the scan data to be transferred into the image data than a conversion scale like a linear scale or the like does. In this regard, it is to be noted that image data that shall be used for displaying on a display means typically only comprises a certain range of gray scale depth (e.g., 256 different gray colors within a true depth color system of 24 bit).

While, in this example, the intensity range of relevance (for e.g., the vitreous body) is mapped to one or even no gray color when using the conventional conversion scale, this range of relevance can be mapped to, e.g., a plurality (e.g., three or four) gray colors. Since the remaining range of intensity (outside the selected range) is still converted using the conventional conversion range, the visibility of the remaining tissues of interest in the subject is affected only at a minimum.

The range of interest can be selected based on the tissue material of interest and its typical intensities in an OCT scan. This range can be obtained, e.g., by means of simulation and/or test measurements.

A main advantage of this different conversion scale for a selected range of intensity data is, besides allowing visualization on display means, an intraoperative use, i.e., the tissue material of interest like the vitreous body of an eye of a patient undergoing a surgery can be visualized on display means without any relevant time delay during the surgery. This allows very exact handling of a surgeon without (toxic) staining the vitreous bod of the eye of the patient.

The invention also relates to an optical coherence tomography (OCT) imaging system for real-time imaging of a subject, e.g. an eye, comprising the control system according to the invention and as described above, and optical coherence tomography imaging means in order to perform the OCT scan (for a more detailed description of such OCT imaging means it is referred to the drawings and the corresponding description). Preferably, the OCT imaging system is configured to visualize the image data on display means. Such display means can be part of the OCT imaging system.

It is of advantage if the OCT imaging system is configured to perform spectral domain optical coherence tomography (abbreviation: SD-OCT). Spectral domain OCT is a kind of frequency domain OCT (FD-OCT) in which broadband interference is acquired with spectrally separated detectors. Another, different kind of frequency domain OCT is swept source OCT (SS-OCT). Swept source OCT encodes the optical frequency in time with a spectrally scanning source. Spectral domain OCT, in contrast, uses a dispersive detector, like a grating and a linear detector array, to separate the different wavelengths. Due to the Fourier relation the depth scan can be immediately calculated by a Fourier-transform from the acquired spectra, without movement of the reference arm. This feature improves imaging speed dramatically, while the reduced losses during a single scan improve the signal to noise ratio proportional to the number of detection elements.

Further, the OCT imaging system is, preferably, configured for use during a surgical procedure being performed on the subject like an eye of a patient. As mentioned before, the specific conversion scale used within the present invention allows an intraoperative use, i.e., the tissue material of interest like the vitreous body of the eye can be visualized on display means without any relevant time delay during the surgery.

The invention also relates to a method for imaging a subject like an eye in real-time, the subject including a tissue material of interest like a vitreous body of an eye, using optical coherence tomography (OCT), preferably, spectral domain OCT. The method comprises the following steps: the subject is scanned by means of OCT and intensity scan data is acquired, the intensity scan data is then processed to provide image data to be visualized on display means. Within these steps, a range of intensity, correlated to the tissue material of interest, is selected, wherein, in the selected range of intensity, the image data is determined based on a selected conversion scale, and wherein, outside the selected range, the image data is determined based on a conversion scale that is different from the selected conversion scale. In a further step, the image data can be visualized on display means.

With respect to further preferred details and advantages of the OCT imaging system and the method, it is also referred to the remarks for the control system above, which apply here correspondingly.

The invention also relates to a computer program with a program code for performing a method according to the invention when the computer program is run on a processor or on a control system according to the invention.

Further advantages and embodiments of the invention will become apparent from the description and the appended figures.

It should be noted that the previously mentioned features and the features to be further described in the following are usable not only in the respectively indicated combination, but also in further combinations or taken alone, without departing from the scope of the present invention.

### Short Description of the Figures

- Fig. 1: shows a schematic overview of an OCT imaging system according to the invention in a preferred embodiment.
- Fig. 2: shows, schematically, different conversion scales as used within the invention, in a diagram.
- Fig. 3: shows, schematically, a flow scheme describing a method according to the invention in a preferred embodiment.

### Detailed Description

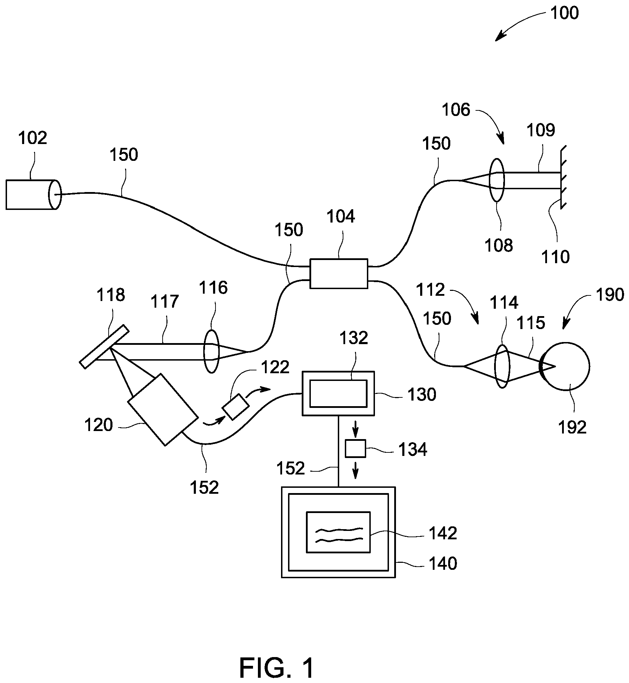

In Fig. 1, a schematic overview of an optical coherence tomography (OCT) imaging system 100 according to the invention in a preferred embodiment is shown. The OCT imaging system 100 comprises a light source 102 (e.g., a low coherence light source), a beam splitter 104, a reference arm 106, a sample arm 112, a diffraction grating 118, a detector 120 (e.g., a camera), a control system 130 and display means 140 (e.g., a display or monitor).

Light originating from the light source 102 is guide, e.g., via fiber optic cables 150, to the beam splitter 104 and a first part of the light is transmitted through the beam splitter 104 and is then guided, via a lens 108 (which is only schematically shown and may represent also different, appropriate optics) in order to create a light beam 109 to a reference mirror 110, wherein the lens 106 and the reference mirror 110 are part of the reference arm 106.

Light reflected from the reference mirror 110 is guided back to the beam splitter 104 and is transmitted through the beam splitter 104 and is then guided, via a lens 116 (which is only schematically shown and may represent also different, appropriate optics) in order to create a light beam 117 to the diffraction grating 118.

A second part of the light, originating from the light source 102 and transmitted through the beam splitter 104 and is guided, via a lens 114 (which is only schematically shown and may represent also different, appropriate optics) in order to create a light beam 115 (for scanning) to the subject 190 to be imaged and which comprises tissue material of interest 192. The lens 114 is part of the sample arm 112.

Light reflected from the subject 190 or the tissue material of interest 192 is guided back to the beam splitter 104 and is transmitted through the beam splitter 104 and is then guided, via lens 116 to the diffraction grating 118. Thus, light reflected in the reference arm 106 and light reflected in the sample arm 112 are combined by means of the beam splitter 104 and are guided, e.g., via a fiber optic cable 150, and in a combined light beam 117 to the diffraction grating 118.

Light reaching the diffraction grating 118 is diffracted and captured by the detector 120. In this way, the detector 120, which acts as a spectrometer, creates or acquires intensity scan data 122 that are transmitted, e.g., via an electrical cable 152, to the control system 130 comprising processing means (or a processor) 132. The intensity scan data 122 is then processed to obtain image data 134 that are, e.g., via an electrical cable 152, to the display means 140 and displayed as a real-time image 142, i.e., an image that represents the currently scanned subject 190 in real-time.

The process in which the intensity scan data 122 is processed or converted to the image data 134 that allows displaying of the scanned subject 190 on the display means 140 will be described in more detail in the following.

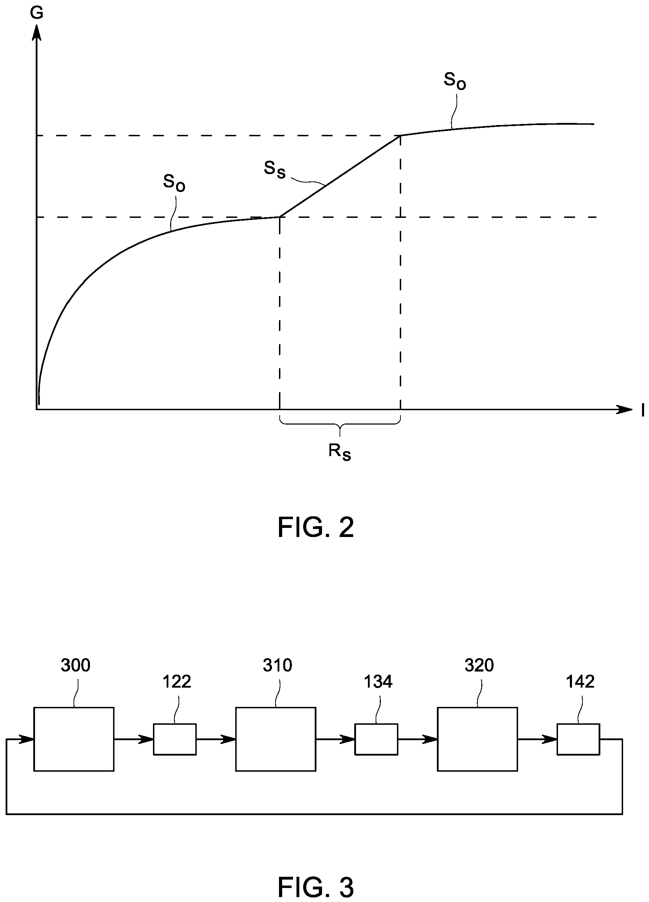

In Fig. 2, different conversion scales as used within the invention, are schematically shown in a diagram. In the diagram, a gray scale value G is shown versus an intensity or intensity value I. Note that while intensity I, typically, comprises continuous values, gray scale values are, typically discrete values or they are continuous and will be displayed in the display means with discrete values.

An exemplary (standard) conversion scale as typically used in OCT or SD-OCT imaging is denoted S_{O}, starting at intensity I=0. This conversion scale S_{O} is based on a logarithmic scale. For example, intensity values I=0 to I=100 are mapped to a gray scale value G=1, intensity values I=100 to I=10^4 are mapped to gray scale value G=2, intensity values I=10^4 to I=10^6 are mapped to gray scale value G=2, and so on.

Such a (standard) conversion scale as it is, up to now, used throughout all intensity values and indicated with dashed line in Fig. 2 results in that a certain range of intensity as, e.g., range R_{S} being mapped to a single gray scale value or being part of a single gray scale value within an even much broader range (which may result in, effectively, no gray scale value).

Within the present invention, such (standard) conversion scale is not used throughout all intensity values but for a selected range of intensity, as indicated with R_{S} in Fig. 2, another and different conversion scale is used. In the example shown in Fig. 2, a conversion scale S_{S} based on a linear scale is used. This different conversion scale S_{S} results in that the intensity values within selected range R_{S} are mapped to a plurality of gray scale values or at least more than for the (standard) conversion scale S_{O}.

For intensity values above the selected range R_{S} the (standard) conversion scale S_{O} is used again. As can be seen in Fig. 2, it shifted with respect to the conversion scale indicated by the dashed line to higher gray scale values G. In other words, the (standard) conversion scale S_{O} is used for the intensity (or all intensity values) outside the selected range R_{S}.

The effect of this different conversion scale S_{S} is that the intensity values within the selected range R_{S} can be made visible in an image that is based on the gray scale values. Thus, the selected range R_{S} can be selected with respect to a tissue material of interest like the vitreous body of an eye as described above. This selected range, i.e., its upper and lower limits, can be selected based upon simulations and/or test measurements.

For example, the intensities obtained from scanning a vitreous body (or any other tissue material of interest) can be measured. The selected range R_{S} can then be adapted accordingly, such that when using the OCT imaging system the intensity values within the selected range R_{S} are automatically processed with the different conversion scale S_{S} and a corresponding real-time image is produced.

It is to be noted that also two or more of such selected ranges with conversion scales different from the (standard) conversion scale can be used if, e.g., to different tissue materials of interest shall be visualized. If necessary, different conversion scales for the different selected ranges can be used. Also, two or more of such selected ranges (with, e.g., the same conversion scale) can be used if the tissue material of interest provides intensities better divided into two or more ranges, wherein intensities between two of these ranges being used with the (standard) conversion scale.

In Fig. 3 a flow scheme describing a method according to the invention in a preferred embodiment using, e.g., the OCT imaging system shown in Fig. 1 is shown. In a first step 300, the subject is scanned by means of OCT as described with respect to Fig. 1 and intensity scan data 122 is acquired and transmitted to the control system with its processing means.

In a next step 310, the intensity scan data is processed by means of the processing means of the control system such that image data 134 is obtained. During this step, intensity values in the intensity scan data are converted into, e.g., grey scale values (as the image data) which can easily be visualized using the conversion scales as shown in and described with respect to Fig. 2. This includes that a range of intensity, correlated to the tissue material of interest, is selected, wherein, in the selected range of intensity, the image data 134 is determined based on the selected conversion scale, and wherein, outside the selected range, the image data is determined based on a conversion scale that is different from the selected conversion scale, e.g., a standard conversion scale as described with respect to Fig. 2.

In a next step 320, the image data 134 is transmitted to and visualized by means of the display means as described with respect to Fig. 1. The result is a real-time image 142 presented by the display means. These steps can be repeatedly or continuously performed such that the real-time image is repeatedly updated or is a live image of the subject showing any changes in the subject in real-time.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to an OCT imaging system comprising a control system as described in connection with one or more of the Figs. 1 to 3. Alternatively, an OCT imaging system may be part of or connected to a system as described in connection with one or more of the Figs. 1 to 3. Fig. 1 shows a schematic illustration of an OCT imaging system 100 configured to perform a method described herein. The OCT imaging system 100 comprises an OCT system and a computer or control system 130. The OCT imaging system 100 is configured to take images and is connected to the computer system 130. The computer system 130 is configured to execute at least a part of a method described herein. The computer system 130 may be configured to execute a machine learning algorithm. The computer system 130 and OCT imaging system 100 may be separate entities but can also be integrated together in one common housing. The computer system 130 may be part of a central processing system of the OCT imaging system 100 and/or the computer system 130 may be part of a subcomponent of the OCT imaging system 100, such as a sensor, an actor, a camera or an illumination unit, etc. of the OCT imaging system 100.

The computer system 130 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 130 may comprise any circuit or combination of circuits. In one embodiment, the computer system 130 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 130 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 130 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 130 may also include a display device, one or more speakers, and a keyboard and/or controller, which can include a mouse, trackball, touch screen, voice-recognition device, or any other device that permits a system user to input information into and receive information from the computer system 130.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of Reference Signs

- 100: OCT imaging system
- 102: light source
- 104: a beam splitter
- 106: reference arm
- 108, 114, 116: lenses
- 109, 115, 117: light beams
- 110: reference mirror
- 112: sample arm
- 118: diffraction grating
- 120: detector
- 122: intensity scan data
- 130: control system
- 132: processing means
- 134: image data
- 140: display means
- 142: real-time image
- 150: fiber optic cable
- 152: electrical cable
- 190: subject
- 192: tissue material of interest

- R_{S}: selected range
- S_{S}: selected conversion scale
- S_{O}: conversion scale

- 300, 310, 320: method steps

## Claims

1. A control system (130) for an optical coherence tomography imaging system (100) for real-time imaging of a subject (190), the subject (190) including a tissue material of interest (192), the control system (130) being configured to:
control the optical coherence tomography imaging system (100) to scan the subject (190) by means of optical coherence tomography for acquiring intensity scan data (122), and
process the intensity scan data (122) to provide image data (134) to be visualized on display means (140), the control system being **characterized in that** for a selected range (R_{S}) of intensity (I), correlated to the tissue material of interest (192), the image data (134) is determined based on a selected conversion scale (S_{S}) that is different from a conversion scale (S_{O}) for intensity outside the selected range (R_{S}).

2. The control system (130) of claim 1, wherein the subject (190) includes or is an eye, and wherein the tissue material of interest (192) includes or is a vitreous body of the eye.

3. The control system (130) of claim 1 or 2, the selected conversion scale (S_{S}) being based on a linear scale.

4. The control system (130) of any one of the preceding claims, the conversion scale (S_{O}) for intensity outside the selected range being based on a logarithmic scale.

5. The control system (130) of any one of the preceding claims, wherein scanning the subject (190) includes performing a B-scan.

6. An optical coherence tomography imaging system (100) for real-time imaging of a subject (190), comprising the control system (130) of any one of the preceding claims and optical coherence tomography imaging means (102, 104, 106, 108, 110, 112, 114, 116, 118, 120).

7. The optical coherence tomography imaging system (100) of claim 6, further configured to visualize the image data (134) on display means (140).

8. The optical coherence tomography imaging system (100) of claim 6 or 7, configured to perform spectral domain optical coherence tomography.

9. The optical coherence tomography imaging system (100) of any one of claims 6 to 8, configured for use during a surgical procedure being performed on the subject (190).

10. A method for imaging a subject (190) in real-time, the subject including a tissue material of interest (192), using optical coherence tomography, comprising the following steps:
scanning the subject (190) by means of optical coherence tomography and acquiring intensity scan data (122), and
processing the intensity scan data (122) to provide image data (134) to be visualized on display means (140),
**characterized in that** the following steps are also included: a range (R_{S}) of intensity, correlated to the tissue material of interest (192), is selected, in the selected range (R_{S}) of intensity (I), the image data (134) is determined based on a selected conversion scale (S_{S}), and outside the selected range, the image data (134) is determined based on a conversion scale (S_{O}) that is different from the selected conversion scale.

11. The method of claim 10, wherein the subject (190) includes or is an eye, and wherein the tissue material of interest (192) includes or is a vitreous body of the eye.

12. The method of claim 10 or 11, the selected conversion scale (S_{S}) being based on a linear scale, and/or the conversion scale (S_{O}) for intensity outside the selected range being based on a logarithmic scale.

13. The method of any one of claims 10 to 12, further comprising the step: visualizing the image data (134) on display means (140).

14. The method of any one of claims 10 to 13, using spectral domain optical coherence tomography.

15. A computer program with a program code for performing the method of any one of claims 10 to 13, when the computer program is run on a processor (132) or on the control system (130) of any one of claims 1 to 5.

## Patentansprüche

1. Steuerungssystem (130) für ein optisches Kohärenztomographie-Abbildungssystem (100) zur Echtzeitabbildung eines Gegenstands (190), wobei der Gegenstand (190) ein Gewebematerial von Interesse (192) einschließt, wobei das Steuerungssystem (130) konfiguriert ist, um:
das optische Kohärenztomographie-Abbildungssystem (100) zu steuern, um den Gegenstand (190) mittels optischer Kohärenztomographie abzutasten, um Intensitätsabtastdaten (122) zu erlangen, und
die Intensitätsabtastdaten (122) zu verarbeiten, um Bilddaten (134) bereitzustellen, die auf Anzeigemitteln (140) visualisiert werden sollen, wobei das Steuerungssystem **dadurch gekennzeichnet ist, dass**
für einen ausgewählten Bereich (R_{S}) der Intensität (I), der mit dem Gewebematerial von Interesse (192) korreliert, die Bilddaten (134) basierend auf einer ausgewählten Umrechnungsskala (S_{S}), die sich von einer Umrechnungsskala (S_{O}) für Intensität außerhalb des ausgewählten Bereichs (R_{S}) unterscheidet, bestimmt werden.

2. Steuerungssystem (130) nach Anspruch 1, wobei der Gegenstand (190) ein Auge einschließt oder ist und wobei das Gewebematerial von Interesse (192) einen Glaskörper des Auges einschließt oder ein solcher ist.

3. Steuerungssystem (130) nach Anspruch 1 oder 2, wobei die ausgewählte Umrechnungsskala (S_{S}) auf einer linearen Scala basiert ist.

4. Steuerungssystem (130) nach einem der vorstehenden Ansprüche, wobei die Umrechnungsskala (S_{O}) für Intensität außerhalb des ausgewählten Bereichs auf einer logarithmischen Skala basiert ist.

5. Steuerungssystem (130) nach einem der vorstehenden Ansprüche, wobei das Abtasten des Gegenstands (190) ein Durchführen einer B-Abtastung einschließt.

6. Optisches Kohärenztomographie-Abbildungssystem (100) zur Echtzeitabbildung eines Gegenstands (190), umfassend ein Steuerungssystem (130) nach einem der vorstehenden Ansprüche und optische Kohärenztomographie-Abbildungsmittel (102, 104, 106, 108, 110, 112, 114, 116, 118, 120).

7. Optisches Kohärenztomographie-Abbildungssystem (100) nach Anspruch 6, das weiter konfiguriert ist, um Bilddaten (134) auf Anzeigemitteln (140) zu visualisieren.

8. Optisches Kohärenztomographie-Abbildungssystem (100) nach Anspruch 6 oder 7, das konfiguriert ist, um optische Spektral-Domänen-Kohärenztomographie durchzuführen.

9. Optisches Kohärenztomographie-Abbildungssystem (100) nach einem der Ansprüche 6 bis 8, das zur Verwendung während einer chirurgischen Prozedur, die an dem Gegenstand (190) durchgeführt wird, konfiguriert ist.

10. Verfahren zum Abbilden eines Gegenstands (190) in Echtzeit unter Verwendung von optischer Kohärenztomographie, wobei der Gegenstand (190) ein Gewebematerial von Interesse (192) einschließt, umfassend die folgenden Schritte:
Abtasten des Gegenstands (190) mittels optischer Kohärenztomographie und Erlangen von Intensitätsabtastdaten (122), und
Verarbeiten der Intensitätsabtastdaten (122), um Bilddaten (134) bereitzustellen, die auf Anzeigemitteln (140) visualisiert werden sollen, **dadurch gekennzeichnet, dass** die folgenden Schritte auch eingeschlossen sind:
Auswählen eines Bereich (R_{S}) der Intensität, der mit dem Gewebematerial von Interesse (192) korreliert,
Bestimmen, in dem ausgewählten Bereich (R_{S}) der Intensität (I), der Bilddaten (134) basierend auf einer ausgewählten Umrechnungsskala (S_{S}), und
Bestimmen, außerhalb des ausgewählten Bereichs, der Bilddaten (134) basierend auf einer Umrechnungsskala (S_{O}), die sich von der ausgewählten Umrechnungsskala unterscheidet.

11. Verfahren nach Anspruch 10, wobei der Gegenstand (190) ein Auge einschließt oder ist und wobei das Gewebematerial von Interesse (192) einen Glaskörper des Auges einschließt oder ein solcher ist.

12. Verfahren nach Anspruch 10 oder 11, wobei die ausgewählte Umrechnungsskala (S_{S}) auf einer linearen Scala basiert ist und/oder die Umrechnungsskala (S_{O}) für Intensität außerhalb des ausgewählten Bereichs auf einer logarithmischen Skala basiert ist.

13. Verfahren nach einem der Ansprüche 10 bis 12, weiter umfassend den Schritt: Visualisieren der Bilddaten (134) auf Anzeigemitteln (140).

14. Verfahren nach einem der Ansprüche 10 bis 13, wobei optische Spektral-Domänen-Kohärenztomographie verwendet wird.

15. Computerprogramm mit einem Programmcode zum Durchführen des Verfahrens nach einem der Ansprüche 10 bis 13, wenn das Computerprogramm auf einem Prozessor (132) oder auf dem Steuerungssystem (130) nach einem der Ansprüche 1 bis 5 laufen gelassen wird.

## Revendications

1. Système de commande (130) pour un système d'imagerie par tomographie par cohérence optique (100) pour l'imagerie en temps réel d'un sujet (190), le sujet (190) incluant un matériau tissulaire d'intérêt (192), le système de commande (130) étant configuré pour :
commander le système d'imagerie par tomographie par cohérence optique (100) pour balayer le sujet (190) au moyen d'une tomographie par cohérence optique pour acquérir des données de balayage d'intensité (122), et
traiter les données de balayage d'intensité (122) pour fournir des données d'image (134) devant être visualisées sur des moyens d'affichage (140), le système de commande étant **caractérisé en ce que** pour une plage sélectionnée (R_{S}) d'intensité (I), corrélée au matériau tissulaire d'intérêt (192), les données d'image (134) sont déterminées sur la base d'une échelle de conversion sélectionnée (S_{S}) qui est différente d'une échelle de conversion (S_{O}) pour l'intensité en dehors de la plage sélectionnée (R_{S}).

2. Système de commande (130) selon la revendication 1, dans lequel le sujet (190) inclut ou est un œil, et dans lequel le matériau tissulaire d'intérêt (192) inclut ou est un corps vitré de l'œil.

3. Système de commande (130) selon la revendication 1 ou 2, l'échelle de conversion sélectionnée (S_{S}) étant basée sur une échelle linéaire.

4. Système de commande (130) selon l'une quelconque des revendications précédentes, l'échelle de conversion (S_{O}) pour l'intensité en dehors de la plage sélectionnée étant basée sur une échelle logarithmique.

5. Système de commande (130) selon l'une quelconque des revendications précédentes, dans lequel le balayage du sujet (190) inclut la mise en œuvre d'un balayage B.

6. Système d'imagerie par tomographie par cohérence optique (100) pour l'imagerie en temps réel d'un sujet (190), comprenant le système de commande (130) selon l'une quelconque des revendications précédentes et des moyens d'imagerie par tomographie par cohérence optique (102, 104, 106, 108, 110, 112, 114, 116, 118, 120).

7. Système d'imagerie par tomographie par cohérence optique (100) selon la revendication 6, en outre configuré pour visualiser les données d'image (134) sur des moyens d'affichage (140).

8. Système d'imagerie par tomographie par cohérence optique (100) selon la revendication 6 ou 7, configuré pour mettre en œuvre une tomographie par cohérence optique de domaine spectral.

9. Système d'imagerie par tomographie par cohérence optique (100) selon l'une quelconque des revendications 6 à 8, configuré pour être utilisé pendant une procédure chirurgicale mise en œuvre sur le sujet (190).

10. Procédé d'imagerie d'un sujet (190) en temps réel, le sujet incluant un matériau tissulaire d'intérêt (192), en utilisant une tomographie par cohérence optique, comprenant les étapes suivantes :
le balayage du sujet (190) par tomographie par cohérence optique et l'acquisition de données de balayage d'intensité (122), et
le traitement des données de balayage d'intensité (122) pour fournir des données d'image (134) devant être visualisées sur des moyens d'affichage (140),
**caractérisé en ce que** les étapes suivantes sont également incluses :
une plage (R_{S}) d'intensité, corrélée au matériau tissulaire d'intérêt (192), est sélectionnée,
dans la plage sélectionnée (R_{S}) d'intensité (I), les données d'image (134) sont déterminées sur la base d'une échelle de conversion sélectionnée (S_{S}), et
en dehors de la plage sélectionnée, les données d'image (134) sont déterminées sur la base d'une échelle de conversion (S_{O}) différente de l'échelle de conversion sélectionnée.

11. Procédé selon la revendication 10, dans lequel le sujet (190) inclut ou est un œil, et dans lequel le matériau tissulaire d'intérêt (192) inclut ou est un corps vitré de l'œil.

12. Procédé selon la revendication 10 ou 11, l'échelle de conversion sélectionnée (S_{S}) étant basée sur une échelle linéaire, et/ou l'échelle de conversion (S_{O}) pour l'intensité en dehors de la plage sélectionnée étant basée sur une échelle logarithmique.

13. Procédé selon l'une quelconque des revendications 10 à 12, incluant en outre l'étape de : visualisation des données d'image (134) sur des moyens d'affichage (140).

14. Procédé selon l'une quelconque des revendications 10 à 13, utilisant la tomographie par cohérence optique de domaine spectral.

15. Programme informatique avec un code de programme pour mettre en œuvre le procédé selon l'une quelconque des revendications 10 à 13, lorsque le programme informatique est exécuté sur un processeur (132) ou sur le système de commande (130) selon l'une quelconque des revendications 1 à 5.
